# FASCICULE DE BREVET EUROPEEN

(11) **EP 0 879 054 B1**
(45) Date de publication et mention de la délivrance du brevet: **28.11.2007**
(21) Numéro de dépôt: 97904476.5
(22) Date de dépôt: 03.02.1997
(51) Int. Cl.: A61K 31/425, A61K 31/40, A61P 25/30

(54) **APPLICATION DE DERIVES DE PYRROLIDINE A LA PREPARATION DE MEDICAMENTS DESTINES AU TRAITEMENT DE L'ABUS DE DROGUES**
VERWENDUNG VON PYROLIDIN-DERIVATEN ZUR HERSTELLUNG VON ARZNEIMITTELN ZUR BEHANDLUNG VON DROGENMISSBRAUCH
APPLICATION OF PYRROLIDINE DERIVATIVES TO THE PREPARATION OF MEDICAMENTS INTENDED TO THE TREATMENT OF DRUG ABUSE

(30) Priorité: 07.02.1996 FR 9601481
(43) Date de publication de la demande: 25.11.1998
(73) Titulaire: Aventis Pharma S.A., 92160 Antony (FR)
(72) Inventeur: BOHME, Andrees, F-75020 Paris (FR); DUBROEUCQ, Marie-Christine, F-95880 Enghien-les-Bains (FR); FRATTA, Walter, I-09124 Cagliari (IT); GUYON, Claude, F-94100 Saint-Maur-des-Fosses (FR); IMPERATO, Assunta, F-75014 Paris (FR); MANFRE, Franco, F-94450 Limeil-Brévannes (FR)
(74) Mandataire: Monain, Patrice
(86) Numéro de dépôt international: PCT/FR1997/000209
(87) Numéro de publication internationale: WO 1997/028798

(56) Documents cités:
- WO-A-93/01167
- WO-A-93/12791
- WO-A-94/15914
- WO-A-94/15954
- WO-A-94/15955
- WO-A-96/38139
- EUROPEAN JOURNAL OF PHARMACOLOGY, vol. 307, no. 3, 1996, pages 283-289, XP000607452 L. SINGH ET AL.: "MODULATION OF THE IN VIVO ACTIONS OF MORPHINE BY THE MIXED CCKA/B RECEPTOR ANTAGONIST PD142898"
- DRUG AND ALCOHOL DEPENDENCE, vol. 34, no. 2, 1994, pages 105-111, XP002017387 B.W. MASSEY ET AL.: "EFFECTS OF CHOLECYSTOKININ ANTAGONISTS ON THE DISCRIMINATIVE STIMULUS EFFECTS OF COCAINE IN RATS AND MONKEYS"
- ARZNEIMITTEL-FORSCHUNG, vol. 42, no. 2A, 1992, pages 250-255, XP000608349 J. HUGHES ET AL.: "NEUROPEPTIDES"

## Description

De nos jours, la dépendance aux drogues, les pharmacomanies et, plus généralement, l'abus de substances licites ou illicites est un problème majeur dans le monde et des produits permettant de diminuer ou de supprimer ces comportements sont devenus nécessaires.

Il a maintenant été trouvé, et c'est ce qui fait l'objet de la présente demande, des composés permettant de traiter l'abus de drogues ou de substances donnant lieu à des pharmacomanies ou à un usage excessif c'est-à-dire des composés qui diminuent ou suppriment la prise de ces produits. Parmi ces drogues et substances donnant lieu à des pharmacomanies ou à un usage excessif, on peut citer la nicotine, les benzodiazépines, la caféine, les stupéfiants tels que amphétamines, cocaïne, cannabinoïdes, morphine et dérivés et opioïdes, les hallucinogènes tels que LSD, ecstasy, mescaline, psylocibine et, en général, tous les composés dont l'abus pose un problème de santé publique.

La présente invention concerne l'utilisation de dérivés de formule: 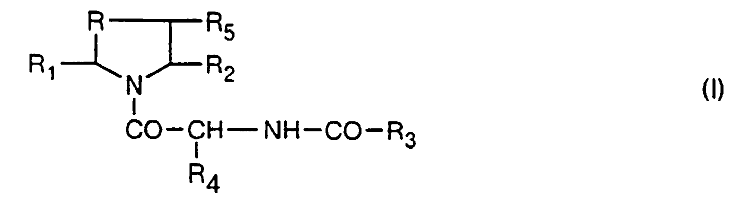 leurs racémiques et énantiomères lorsqu'il contiennent un ou plusieurs centres asymétriques et leurs sels au traitement de l'abus de drogues ou de substances donnant lieu à des pharmacomanies ou à un usage excessif à l'exception de l'abus d'alcool par diminution de l'auto administration desdites drogues par le patient.

La présente invention concerne également l'utilisation de ces composés à la préparation d'un médicament destiné au traitement de l'abus de drogues ou de substances donnant lieu à des pharmacomanies ou à un usage excessif à l'exception de l'abus d'alcool.

Dans la formule (I),
- soit R représente un radical méthylène, éthylène, SO, SO₂, CHOH ou un atome de soufre, R₁ représente un radical pyridyle éventuellement substitué par un ou plusieurs radicaux alkyle, furyle éventuellement substitué par un ou plusieurs radicaux alkyle, thiènyle éventuellement substitué par un ou plusieurs radicaux alkyle, quinolyle éventuellement substitué par un ou plusieurs radicaux alkyle, naphtyle éventuellement substitué par un ou plusieurs radicaux alkyle, indolyle éventuellement substitué par un ou plusieurs radicaux alkyle ou phényle éventuellement substitué par un ou plusieurs substituants choisis parmi les atomes d'halogène et les radicaux alkyle, alcoxy, hydroxy, nitro, amino, monoalkylamino, dialkylamino, alcoxycarbonyle, -CO-NR₇R₈, -NH-CO-CH₃, trifluorométhyle ou trifluorométhoxy et R₅ représente un atome d'hydrogène,
- soit R représente un radical méthylène, R₁ représente un atome d'hydrogène et R₅ représente un radical phényle,
- soit R représente un radical CHR₆, R₁ et R₅ représentent chacun un atome d'hydrogène,
- R₂ représente un radical alcoxycarbonyle, cycloalkyloxycarbonyle, cycloalkylalkyloxycarbonyle, -CONR₉R₁₀ ou phényle éventuellement substitué par un ou plusieurs substituants choisis parmi les radicaux alkyle, alcoxy ou hydroxy,
- R₃ représente un radical phényle (éventuellement substitué par un ou plusieurs substituants choisis parmi les atomes d'halogène et les radicaux alkyle, alcoxy et alkylthio), naphtyle, indolyle, quinolyle ou phénylamino dont le noyau phényle est éventuellement substitué par un ou plusieurs substituants choisis parmi les atomes d'halogène et les radicaux alkyle, alcoxy, alkylthio, trifluorométhyle, carboxy, alcoxycarbonyle, hydroxy, nitro, amino, acyle, cyano, sulfamoyle, carbamoyle, hydroxyiminoalkyle, alcoxyiminoalkyle, hydroxyaminocarbonyle, alcoxyaminocarbonyle, tétrazolyl-5, tétrazolyl-5 alkyle, trifluorométhylsulfonamido, alkylsulfinyle, mono ou polyhydroxyalkyle, sulfo, -alk-O-CO-alk, -alk-COOX, -alk-O-alk, -alk'-COOX, -O-alk-COOX, -CH=CH-COOX, -CO-COOX, -alk-SO₃H, sous forme de sel, -CH=CH-alk', -C(=NOH)-COOX, -S-alk-COOX, -O-CH₂-alk'-COOX, -CX=N-O-alk-COOX, -alk-N(OH)-CO-alk ou diméthyl-2,2 dioxo-4,6 dioxanne-1,3-yle-5,
- R₄ représente un atome d'hydrogène ou un radical alkyle,
- R₆ représente un radical phényle,
- R₇ représente un atome d'hydrogène ou un radical alkyle, phénylalkyle ou phényle éventuellement substitué par un ou plusieurs substituants choisis parmi les atomes d'halogène et les radicaux alkyle, alcoxy et alkylthio,
- R₈ représente un radical alkyle, phénylalkyle ou phényle éventuellement substitué par un ou plusieurs substituants choisis parmi les atomes d'halogène et les radicaux alkyle, alcoxy et alkylthio,
   ou bien R₇ et R₈ forment avec l'atome d'azote auquel ils sont rattachés un hétérocycle mono ou polycyclique saturé ou insaturé contenant 4 à 9 atomes de carbone et un ou plusieurs hétéroatomes (O, N) et éventuellement substitué par un ou plusieurs radicaux alkyle,
- R₉ représente un atome d'hydrogène ou un radical alkyle, cycloalkylalkyle, cycloalkyle, phénylalkyle ou phényle éventuellement substitué par un ou plusieurs substituants choisis parmi les atomes d'halogène et les radicaux alkyle, alcoxy et alkylthio,
- R₁₀ représente un radical alkyle, cycloalkylalkyle, cycloalkyle, phénylalkyle ou phényle éventuellement substitué par un ou plusieurs substituants choisis parmi les atomes d'halogène et les radicaux alkyle, alcoxy et alkylthio,
   ou bien R₉ et R₁₀ forment ensemble avec l'atome d'azote auquel ils sont rattachés un hétérocycle mono ou polycyclique saturé ou insaturé contenant 4 à 9 atomes de carbone et un ou plusieurs hétéroatomes (O, N, S) et éventuellement substitué par un ou plusieurs radicaux alkyle,
- X représente un atome d'hydrogène, un radical alkyle ou phénylalkyle,
- alk représente un radical alkyle ou alkylène,
- alk' représente un radical hydroxyalkyle, hydroxyalkylène, alkoxyalkyle ou alkoxyalkylène.

Dans les définitions qui précèdent et celles qui seront citées ci-après, sauf mention contraire, les radicaux alkyle, alkylène et alcoxy et les portions alkyle, alkylène et alcoxy contiennent 1 à 4 atomes de carbone en chaîne droite ou ramifiée, les radicaux ou portions acyle contiennent 2 à 4 atomes de carbone et les radicaux et portions cycloalkyle contiennent 3 à 6 atomes de carbone.

Lorsque R₇ et R₈ forment avec l'atome d'azote auquel ils sont rattachés un hétérocycle, celui-ci est de préférence un cycle pipéridino éventuellement substitué par un ou plusieurs radicaux alkyle ou un cycle tétrahydro-1,2,3,4 quinoléine.

Lorsque R₉ et R₁₀ forment avec l'atome d'azote auquel ils sont rattachés un hétérocycle, celui-ci est de préférence un cycle pipéridino, perhydroazépinyl-1, tétrahydro-1,2,3,6 pyridyl-1, tétrahydro-1,2,3,4 quinolyl-1, pyrrolidinyl-1, tétrahydro-1,2,3,4 isoquinolyl-2, thiomorpholino ou indolyl-1, ces cycles pouvant être éventuellement substitués par au moins un radical alkyle.

Les composés de formule (I) comportant un ou plusieurs centres asymétriques présentent des formes isomères. Les racémiques et les énantiomères de ces composés font également partie de l'invention.

Les composés de formule (I) peuvent éventuellement exister sous forme de sels d'addition avec un acide minéral ou organique.

Les composés de formule (1) comportant un reste carboxy, sulfo ou alk-SO₃H peuvent également exister sous forme de sels métalliques ou de sels d'addition avec les bases azotées pharmaceutiquement acceptables.

Comme exemples de sels pharmaceutiquement acceptables, peuvent être cités les sels d'addition avec les acides minéraux ou organiques (tels que acétate, propionate, succinate, benzoate, fumarate, maléate, oxalate, méthanesulfonate, iséthionate, théophyllinacétate, salicylate, méthylène-bis-β-oxynaphtoate, chlorhydrate, sulfate, nitrate et phosphate), les sels avec les métaux alcalins (sodium, potassium, lithium) ou avec les métaux alcalinoterreux (calcium, magnésium), le sel d'ammonium, les sels de bases azotées (éthanolamine, triméthylamine, méthylamine, benzylamine, N-benzyl-β-phénéthylamine, choline, arginine, leucine, lysine, N-méthyl glucamine).

Les composés de formule (I) et leurs sels peuvent être préparés dans les conditions décrites dans la demande internationale WO 93/01167.

D'après la demande internationale WO 93/01167, les composés de formule (I) présentent des propriétés pharmacologiques intéressantes. Ces composés possèdent une forte affinité pour les récepteurs de la cholécystokinine (CCK) et de la gastrine et sont donc utiles dans le traitement et la prévention des désordres liés à la CCK et à la gastrine au niveau du système nerveux et de l'appareil gastrointestinal.

C'est ainsi que selon la demande internationale PCT WO 93/01167, les composés peuvent être utilisés pour le traitement ou la prévention des psychoses, des troubles anxieux, de la maladie de Parkinson, de la diskinésie tardive, du syndrôme du colon irritable, de la pancréatite aiguë, des ulcères, des désordres de la motilité intestinale, de certaines tumeurs sensibles à la CCK et comme régulateur de l'appétit. Ces composés, qui ont également un effet de potentialisation sur l'activité analgésique des médicaments narcotiques et non narcotiques, peuvent avoir un effet analgésique propre. Par ailleurs, les composés qui ont une forte affinité pour les récepteurs CCK modifient les capacités de mémorisation, peuvent être efficaces dans les troubles de la mémoire.

L'effet des composés de formule (I) pour le traitement de l'abus de drogues ou de substances donnant lieu à des pharmacomanies ou à un usage excessif a été évalué dans le test de l'auto-administration de drogues chez la souris selon le protocole qu'a décrit A. KUZMIN et coll., Pharmacol. Biochem. Behav., 41, 497-500 (1992) pour la morphine et la cocaïne. Dans ce test, les composés de formule (I), à des doses égales ou inférieures à 100 mg/kg, s'opposent à l'auto-administration de drogues ou de substances donnant lieu à des pharmacomanies ou à un usage excessif (amphétamine, cocaïne, morphine, diazépam, mescaline).

D'un intérêt particulier sont les composés de formule (I) pour lesquels R représente un radical méthylène, un atome de soufre ou un radical SO, R₁ représente un radical phényle éventuellement substitué, R₂ représente un radical phényle ou alcoxycarbonyle, R₄ et R₅ représente un atome d'hydrogène, R₃ représente un radical phénylamino dont le noyau phényle est substitué par un radical carboxy, -alk-COOH, -S-alk-COOH, hydroxyalkyle, alk'-COOH ou alkSO₃H, hydroxyiminoalkyle, leurs racémiques et énantiomères lorsqu'il contiennent un ou plusieurs centres asymétriques et leurs sels. Plus particulièrement intéressants sont les produits de formule (I) dans laquelle R₁ et R₂ sont en position cis l'un par rapport à l'autre.

D'un intérêt particulier sont les composés suivants :
- {[(hydroxy-1 éthyl-(RS))-3 phényl)-3 uréido]-2 acétyl}-1 phényl-5 prolinate de tert-butyle-(2RS,5SR),
- acide {{[tert-butoxycarbonyl-2 phényl-5 pyrrolidinyl-1 (2S,5R))-2 oxo-2 éthyl]-3 uréido}-3 phényl}-2 propionique,
- acide {{[(tert-butoxycarbonyl-2 phényl-5 pyrrolidinyl-1)-2 oxo-2 éthyl]-3 uréido}-3 phénylthio} acétique-(2RS,5SR),
- acide {{[(tert-butoxycarbonyl-4 (fluoro-2 phényl)-2 thiazolidinyl-3-(2R,4R))-2 oxo-2 éthyl]-3 uréido}-3 phényl}-2 propionique,
- {{[(tert-butoxycarbonyl-4 phényl-2 thiazolidinyl-3-(2R,4R)))-2 oxo-2 éthyl]-3 uréido}-3 phényl}-1 éthanesulfonate de potassium-(RS),
- {{[(tert-butoxycarbonyl-2 phényl-5 pyrrolidinyl-1-(2S,5R))-2 oxo-2 éthyl]-3 uréido}-3 phényl}-1 éthanesulfonate de potassium-(RS),
- {{[(tert-butoxycarbonyl-2 phényl-5 pyrrolidinyl-1)-2 oxo-2 éthyl]-3 uréïdo}-3 phényl-1 méthanesulfonate de potassium-(2S,5R).
- acide {[tert-butoxycarbonyl-2 phényl-5 pyrrolidinyl-1)-2 oxo-2 éthyl]-3 uréido}-3 benzoïque-(2S,5R),
- acide {([tert-butoxycarbonyl-2 (fluoro-2 phényl)-5 pyrrolidinyl-1]-2 oxo-2 éthyt}-3 uréidol-3 benzoïque-(2RS,5SR),
- acide {[(diphényl-2,5 pyrrolidinyl-1)-2 oxo-2 éthyl]-3 uréido}-3 benzoïque-(cis),
- acide {{[(hydroxy-2 phényl)-2 phényi-5 pyrrolidinyl-1]-2 oxo-2 uréido}-3 phénylacétique-(2RS,5SR),
- acide {[(tert-butoxycarbonyl-4 phényl-2 thiazolidinyl-3)-2 oxo-2 éthyl]-3 uréido}-3 phénylacétique-(2R,4R),
- acide {[(tert-butoxycarbonyl-4 phényl-2 thiazolidinyl-3)-2 oxo-2 éthyll-3 uréido}-3 benzoique-(2R,4R),
- acide {{[(tert-butoxycarbonyl-4 (fluoro-2 phényl)-2 oxyde-1 thiazolidinyl-3-(1 RS,2R,4R))-2 oxo-2 éthyl}-3 uréidol-3 phényl}-2 propionique,
- acide {[(tert-butoxycarbonyl-4 (difluoro-2,3 phényl)-2 thiazolidinyl-3)-2 oxo-2 éthyl]-3 uréido}-3 phénylacétique-(2R,4R),
- {{[(hydroxyimino-1 éthyl)-3 phényl-(E)]-3 uréido}-2 acétyl}-1 phényl-5 prolinate de tert-butyle-(2RS,5SR)
leurs racémiques et énantiomères lorsqu'ils contiennent un ou plusieurs centres asymétriques et leurs sels.

Les médicaments selon l'invention sont constitués par un composé de formule (I) sous forme libre ou sous forme d'un sel d'addition avec un acide pharmaceutiquement acceptable, à l'état pur ou sous forme d'une composition dans laquelle il est associé à tout autre produit pharmaceutiquement compatible, pouvant être inerte ou physiologiquement actif. Les médicaments selon l'invention peuvent être employés par voie orale, parentérale, rectale ou topique.

Comme compositions solides pour administration orale, peuvent être utilisés des comprimés, des pilules, des poudres (capsules de gélatine, cachets) ou des granulés. Dans ces compositions, le principe actif selon l'invention est mélangé à un ou plusieurs diluants inertes, tels que amidon, cellulose, saccharose, lactose ou silice, sous courant d'argon. Ces compositions peuvent également comprendre des substances autres que les diluants, par exemple un ou plusieurs lubrifiants tels que le stéarate de magnésium ou le talc, un colorant, un enrobage (dragées) ou un vernis.

Comme compositions liquides pour administration orale, on peut utiliser des solutions, des suspensions, des émulsions, des sirops et des élixirs pharmaceutiquement acceptables contenant des diluants inertes tels que l'eau, l'éthanol, le glycérol, les huiles végétales ou l'huile de paraffine. Ces compositions peuvent comprendre des substances autres que les diluants, par exemple des produits mouillants, édulcorants, épaississants, aromatisants ou stabilisants.

Les compositions stériles pour administration parentérale, peuvent être de préférence des solutions aqueuses ou non aqueuses, des suspensions ou des émulsions. Comme solvant ou véhicule, on peut employer l'eau, le propylèneglycol, un polyéthylèneglycol, des huiles végétales, en particulier l'huile d'olive, des esters organiques injectables, par exemple l'oléate d'éthyle ou d'autres solvants organiques convenables. Ces compositions peuvent également contenir des adjuvants, en particulier des agents mouillants, isotonisants, émulsifiants, dispersants et stabilisants. La stérilisation peut se faire de plusieurs façons, par exemple par filtration aseptisante, en incorporant à la composition des agents stérilisants, par irradiation ou par chauffage. Elles peuvent également être préparées sous forme de compositions solides stériles qui peuvent être dissoutes au moment de l'emploi dans de l'eau stérile ou tout autre milieu stérile injectable.

Les compositions pour administration rectale sont les suppositoires ou les capsules rectales qui contiennent, outre le produit actif, des excipients tels que le beurre de cacao, des glycérides semi-synthétiques ou des polyéthylèneglycols.

Les compositions pour administration topique peuvent être par exemple des crèmes, lotions, collyres, collutoires, gouttes nasales ou aérosols.

En thérapeutique humaine, les médicaments selon l'invention sont particulièrement utiles dans le traitement d'abus de drogues ou de substances donnant lieu à des pharmacomanies ou à un usage excessif à l'exception de l'abus d'alcool.

Les doses dépendent de l'effet recherché, de la durée du traitement et de la voie d'administration utilisée; elles sont généralement comprises entre 0,05 g et 1 g par jour par voie orale pour un adulte avec des doses unitaires allant de 10 mg à 500 mg de substance active.

D'une façon générale, le médecin déterminera la posologie appropirée en fonction de l'âge, du poids et de tous les autres facteurs propres au sujet à traiter.

Les exemples suivants illustrent des médicaments selon l'invention :

### EXEMPLE A

On prépare, selon la technique habituelle, des gélules dosées à 50 mg de produit actif ayant la composition suivante :
- Composé de formule (I) 50 mg
- Cellulose 18 mg
- Lactose 55 mg
- Silice colloïdale 1 mg
- Carboxyméthylamidon sodique 10 mg
- Talc 10 mg
- Stéarate de magnésium 1 mg

### EXEMPLE B

On prépare selon la technique habituelle des comprimés dosés à 50 mg de produit actif ayant la composition suivante :
- Composé de formule (I) 50 mg
- Lactose 104 mg
- Cellulose 40 mg
- Polyvidone 10 mg
- Carboxyméthylamidon sodique 22 mg
- Talc 10 mg
- Stéarate de magnésium 2 mg
- Silice colloïdale 2 mg
- Mélange d'hydroxyméthylcellulose, glycérine, oxyde de titane (72-3,5-24,5) q.s.p. 1 comprimé pelliculé terminé à 245 mg

### EXEMPLE C

On prépare une solution injectable contenant 10 mg de produit actif ayant la composition suivante :
- Composé de formule (I) 10 mg
- Acide benzoïque 80 mg
- Alcool benzylique 0,06 cm³
- Benzoate de sodium 80 mg
- Ethanol à 95 % 0,4 cm³
- Hydroxyde de sodium 24 mg
- Propylène glycol 1,6 cm³
- Eau q.s.p. 4 cm3

## Revendications

1. Utilisation des composés de formule (I) ci-après : 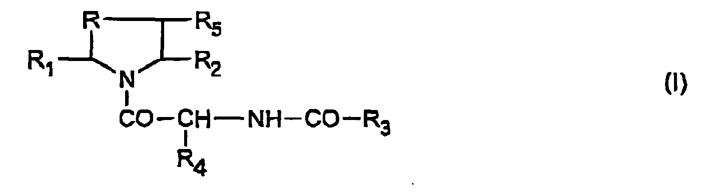 dans laquelle
- soit R représente un radical méthylène, éthylène, SO, SO₂, CHOH ou un atome de soufre, R₁ représente un radical pyridyle éventuellement substitué par un ou plusieurs radicaux alkyle, furyle éventuellement substitué par un ou plusieurs radicaux alkyle, thiènyle éventuellement substitué par un ou plusieurs radicaux alkyle, quinolyle éventuellement substitué par un ou plusieurs radicaux alkyle, naphtyle éventuellement substitué par un ou plusieurs radicaux alkyle, indolyle éventuellement substitué par un ou plusieurs radicaux alkyle ou phényle éventuellement substitué par un ou plusieurs substituants choisis parmi les atomes d'halogène et les radicaux alkyle, alcoxy, hydroxy, nitro, amino, monoalkylamino, dialkylamino, alcoxycarbonyle, -CO-NR₇R₈, -NH-CO-CH₃, trifluorométhyle ou trifluorométhoxy et R₅ représente un atome d'hydrogène,
- soit R représente un radical méthylène, R₁ représente un atome d'hydrogène et R₅ représente un radical phényle,
- soit R représente un radical CHR₆, R₁ et R₅ représentent chacun un atome d'hydrogène,
- R₂ représente un radical alcoxycarbonyle, cycloalkyloxycarbonyle, cycloalkylalkyloxycarbonyle, -CONR₉R₁₀ ou phényle éventuellement substitué par un ou plusieurs substituants choisis parmi les radicaux alkyle, alcoxy ou hydroxy,
- R₃ représente un radical phényle (éventuellement substitué par un ou plusieurs substituants choisis parmi les atomes d'halogène et les radicaux alkyle, alcoxy et alkylthio), naphtyle, indolyle, quinolyle ou phénylamino dont le noyau phényle est éventuellement substitué par un ou plusieurs substituants choisis parmi les atomes d'halogène et les radicaux alkyle, alcoxy, alkylthio, trifluorométhyle, carboxy, alcoxycarbonyle, hydroxy, nitro, amino, acyle, cyano, sulfamoyle, carbamoyle, hydroxyiminoalkyle, alcoxyiminoalkyle, hydroxyaminocarbonyle, alcoxyaminocarbonyle, tétrazolyl-5, tétrazolyl-5 alkyle, triflucrométhylsulfonamido, alkylsulfinyle, mono ou polyhydroxyalkyle, sulfo, -alk-O-CO-alk, -alk-COOX, -alk-O-alk, -alk'-COOX, -O-alk COOX, -CH=CH-COOX. -CO-COOX, -alk-SO₃H, sous forme de sel, -CH=CH-alk'. -C(=NOH)-COOX, -S-alk-COOX, -O-CH₂-alk'-COOX, -CX=N-O-alk-COOX, -alk-N(OH)-CO-alk ou diméthyl-2,2 dioxo-4,6 dioxanne-1,3-yle-5,
- R₄ représente un atome d'hydrogène ou un radical alkyle,
- R₆ représente un radical phényle,
- R₇ représente un atome d'hydrogène ou un radical alkyle, phénylalkyle ou phényle éventuellement substitué par un ou plusieurs substituants choisis parmi les atomes d'halogène et les radicaux alkyle, alcoxy et alkylthio,
- R₈ représente un radical alkyle, phénylalkyle ou phényle éventuellement substitué par un ou plusieurs substituants choisis parmi les atomes d'halogène et les radicaux alkyle, alcoxy et alkylthio,
ou bien R₇ et R₈ forment avec l'atome d'azote auquel ils sont rattachés un hétérocycle mono ou polycyclique saturé ou insaturé contenant 4 à 9 atomes de carbone et un ou plusieurs hétéroatomes (O, N) et éventuellement substitué par un ou plusieurs radicaux alkyle,
- R₉ représente un atome d'hydrogène ou un radical alkyle, cycloalkylalkyle, cycloalkyle, phénylalkyle ou phényle éventuellement substitué par un ou plusieurs substituants choisis parmi les atomes d'halogène et les radicaux alkyle, alcoxy et alkylthio,
- R₁₀ représente un radical alkyle, cycloalkylalkyle, cycloalkyle, phénylalkyle ou phényle éventuellement substitué par un ou plusieurs substituants choisis parmi les atomes d'halogène et les radicaux alkyle, alcoxy et alkylthio,
ou bien R₉ et R₁₀ forment ensemble avec l'atome d'azote auquel ils sont rattachés un hétérocycle mono ou polycyclique saturé ou insaturé contenant 4 à 9 atomes de carbone et un ou plusieurs hétéroatomes- (O, N, S) et éventuellement substitué par un ou plusieurs radicaux alkyle,
- X représente un atome d'hydrogène, un radical alkyle ou phénylalkyle,
- alk représente un radical alkyle ou alkylène,
- alk' représente un radical hydroxyalkyle, hydroxyalkylène, alkoxyalkyle ou alkoxyalkylène,
leurs racémiques et énantiomères lorsqu'ils contiennent un ou plusieurs centres asymétriques et leurs sels,
étant entendu que les radicaux alkyle, alkylène et alcoxy et les portions alkyle, alkylène et alcoxy contiennent 1 à 4 atomes de carbone en chaîne droite ou ramifiée, les radicaux et portions acyle contiennent 2 à 4 atomes de carbone et les radicaux et portions cycloalkyle contiennent 3 à 6 atomes de carbone, ainsi que leurs sels et leurs racémiques et énantiomères lorsqu'ils comportent au moins un centre asymétrique, pour la préparation d'un médicament destiné au traitement de l'abus de drogues ou de substances donnant lieu à des pharmacomanies ou à un usage excessif, à l'exception de l'abus d'alcool, par diminution de l'auto administration desdites drogues par le patient.

2. Utilisation selon la revendication 1, **caractérisée en ce que** R représente un radical méthylène, un atome de soufre ou un radical SO, R₁ représente un radical phényle éventuellement substitué, R₂ représente un radical phényle ou alcoxycarbonyle, R₄ et R₅ représentent un atome d'hydrogène et **R₃** représente un radical phénylamino dont le noyau phényle est substitué par un radical carboxy, -alk-COOH, -S-alk-COOH, hydroxyalkyle, -alk'-COOH ou -alk-SO₃H.

3. Utilisation selon la revendication 1 ou 2, **caractérisée en ce que** R₁ et R₂ sont en position cis l'un par rapport à l'autre.

4. Utilisation selon la revendication 1, **caractérisée en ce que** le composé de formule (I) est choisi parmi :
- {[(hydroxy-1 éthyl-(RS))-3 phényl)-3 uréido]-2 acétyl}-1 phényl-5 prolinate de tert-butyle-(2RS,5SR),
- acide {([(tert-butoxycarbonyl-2 phényl-5 pyrrolidinyl-1 (2S,5R))-2 oxo-2 éthyl]-3 uréido)-3 phényl)-2 propionique,
- acide {{[(tert-butoxycarbonyl-2 phényl-5 pyrrolidinyl-1)-2 oxo-2 éthyl]-3 uréido}-3 phénylthio} acétique-(2RS,5SR),
- acide {{[(tert-butoxycarbonyl-4 (fluoro-2 phényl)-2 thiazolidinyl-3-(2R,4R))-2 oxo-2 éthyl]-3 uréido}-3 phényl}-2 propionique,
- {{[(tert-butoxycarbonyl-4 phényl-2 thiazolidinyl-3-(2R,4R)))-2 oxo-2 éthyl]-3 uréido}-3 phényl}-1 éthanesulfonate de potassium-(RS),
- {{[(tert-butoxycarbonyl-2 phényl-5 pyrrolidinyl-1-(2S,5R))-2 oxo-2 éthyl]-3 uréido}-3 phényl}-1 éthanesulfonate de potassium-(RS),
- {{[(tert-butoxycarbonyl-2 phényl-5 pyrrolidinyl-1)-2 oxo-2 éthyl]-3 uréïdo}-3 phényl-1 méthanesulfonate de potassium-(2S,5R).
- acide {[tert-butoxycarbonyl-2 phényl-5 pyrrolidinyl-1)-2 oxo-2 éthyl]-3 uréido}-3 benzoïque-(2S,5R),
- acide ({[tert-butoxycarbonyl-2 (fluoro-2 phényl)-5 pyrrolidinyl-1]-2 oxo-2 éthyl}-3 uréido}-3 benzoïque-(2RS,5SR),
- acide {[(diphényl-2,5 pyrrolidinyl-1)-2 oxo-2 éthyl]-3 uréido}-3 benzoïque-(cis).
- acide {{[(hydroxy-2 phényl)-2 phényl-5 pyrrolidinyl-1]-2 oxo-2 uréido}-3 phénylacétique-(2RS,5SR),
- acide {[(tert-butoxycarbonyl-4 phényl-2 thiazolidinyl-3)-2 oxo-2 éthyl]-3 uréido}-3 phénylacétique-(2R,4R),
- acide {[(tert-butoxycarbonyl-4 phényl-2 thiazolldinyl-3)-2 oxo-2 éthyl]-3 uréido}-3 benzoïque-(2R,4R),
- acide {{((tert-butoxycarbonyl-4 (fluoro-2 phényl)-2 oxyde-1 thiazolidinyl-3-(1RS,2R,4R))-2 oxo-2 éthyl}-3 urëido}-3 phényl}-2 propionique,
- acide {[(tert-butoxycarbonyl-4 (difluoro-2,3 phényl)-2 thiazolidinyl-3)-2 oxo-2 éthyl]-3 uréido}-3 phénylacétique-(2R,4R),
- {{[(hydroxyimino-1 éthyl)-3 phényl-(E)]-3 uréido}-2 acêtyl}-1 phényl-5 prolinate de tert-butyle-(2RS,5SR).

5. Utilisation selon la revendication 4, **caractérisée en ce que** le composé de formule (I) est l'acide {{[(tert-butoxycarbonyl-4 (fluoro-2 phényl)-2 thiazolidinyl-3-(2R,4R))-2 oxo-2 éthyl]-3 uréido}-3 phényl}-2 propionique.

6. Utilisation selon l'une quelconque des revendications 1 à 5, **caractérisée en ce que** la drogue est choisie parmi la nicotine, la caféine, une benzodiazépine, un stupéfiant ou un hallucinogène.

7. Utilisation selon la revendication 6, **caractérisée en ce que** le stupéfiant est une amphétamine, la cocaïne, un cannabinoïde, la morphine ou un de ses dérivés ou un opioïde.

8. Utilisation selon la revendication 6, **caractérisée en ce que** l'hallucinogène est le LSD, l'ecstasy, la mescaline ou la psylocibine.

## Claims

1. Use of the compounds of formula (I) below: 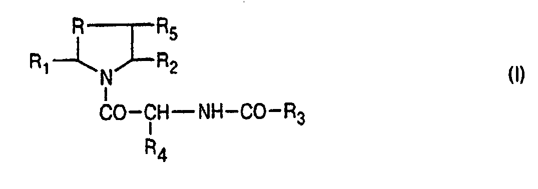 in which
- either R represents a methylene, ethylene, SO, SO₂ or CHOH radical or a sulphur atom, R₁ represents a pyridyl radical optionally substituted by one or more alkyl radicals, a furyl radical optionally substituted by one or more alkyl radicals, a thienyl radical optionally substituted by one or more alkyl radicals, a quinolyl radical optionally substituted by one or more alkyl radicals, a naphthyl radical optionally substituted by one or more alkyl radicals, an indolyl radical optionally substituted by one or more alkyl radicals or a phenyl radical optionally substituted by one or more substituents chosen from halogen atoms and alkyl, alkoxy, hydroxyl, nitro, amino, monoalkylamino, dialkylamino, alkoxycarbonyl, -CO-NR₇R₈, -NH-CO-CH₃, trifluoromethyl or trifluoromethoxy radicals and R₅ represents a hydrogen atom,
- or R represents a methylene radical, R₁ represents a hydrogen atom and R₅ represents a phenyl radical,
- or R represents a CHR₆ radical and R₁ and R₅ each represent a hydrogen atom,
- R₂ represents an alkoxycarbonyl radical, a cycloalkyloxycarbonyl radical, a cycloalkylalkyloxycarbonyl radical, a -CONR₉R₁₀ radical, or a phenyl radical optionally substituted by one or more substituents chosen from alkyl, alkoxy or hydroxyl radicals,
- R₃ represents a phenyl radical (optionally substituted by one or more substituents chosen from halogen atoms and alkyl, alkoxy and alkylthio radicals), a naphthyl radical, an indolyl radical, a quinolyl radical or a phenylamino radical, the phenyl ring of which is optionally substituted by one or more substituents chosen from halogen atoms and alkyl, alkoxy, alkylthio, trifluoromethyl, carboxyl, alkoxycarbonyl, hydroxyl, nitro, amino, acyl, cyano, sulphamoyl, carbamoyl, hydroxyiminoalkyl, alkoxyiminoalkyl, hydroxyaminocarbonyl, alkoxyaminocarbonyl, tetrazol-5-yl, tetrazol-5-ylalkyl, trifluoromethylsulphonamido, alkylsulphinyl, mono- or polyhydroxyalkyl, sulpho, -alk-O-CO-alk, -alk-COOX, -alk-O-alk, -alk'-COOX, -O-alk-COOX, -CH=CH-COOX, -CO-COOX, -alk-SO₃H, in the salt form, -CH=CH-alk', -C(=NOH)-COOX, -S-alk-COOX, -O-CH₂-alk'-COOX, -CX=N-O-alk-COOX, -alk-N(OH)-CO-alk, or 2,2-dimethyl-4,6-dioxo-1,3-dioxan-5-yl radicals,
- R₄ represents a hydrogen atom or an alkyl radical,
- R₆ represents a phenyl radical,
- R₇ represents a hydrogen atom or an alkyl radical, a phenylalkyl radical or a phenyl radical optionally substituted by one or more substituents chosen from halogen atoms and alkyl, alkoxy and alkylthio radicals,
- R₈ represents an alkyl radical, a phenylalkyl radical or a phenyl radical optionally substituted by one or more substituents chosen from halogen atoms and alkyl, alkoxy and alkylthio radicals,
or else R₇ and R₈ form, with the nitrogen atom to which they are attached, a saturated or unsaturated and mono- or polycyclic heterocycle which comprises 4 to 9 carbon atoms and one or more heteroatoms (0, N) and which is optionally substituted by one or more alkyl radicals,
- R₉ represents a hydrogen atom or an alkyl radical, a cycloalkylalkyl radical, a cycloalkyl radical, a phenylalkyl radical or a phenyl radical optionally substituted by one or more substituents chosen from halogen atoms and alkyl, alkoxy and alkylthio radicals,
- R₁₀ represents an alkyl radical, a cycloalkylalkyl radical, a cycloalkyl radical, a phenylalkyl radical or a phenyl radical optionally substituted by one or more substituents chosen from halogen atoms and alkyl, alkoxy and alkylthio radicals,
or else R₉ and R₁₀ form, together with the nitrogen atom to which they are attached, a saturated or unsaturated and mono- or polycyclic heterocycle which comprises 4 to 9 carbon atoms and one or more heteroatoms (O, N, S) and which is optionally substituted by one or more alkyl radicals,
- X represents a hydrogen atom or an alkyl or phenylalkyl radical,
- alk represents an alkyl or alkylene radical,
- alk' represents a hydroxyalkyl, hydroxyalkylene, alkoxyalkyl or alkoxyalkylene radical,
their racemates and enantiomers, when they comprise one or more asymmetric centres, and their salts,
it being understood that the alkyl, alkylene and alkoxy radicals and the alkyl, alkylene and alkoxy portions comprise 1 to 4 straight- or branched-chain carbon atoms, the acyl radicals and portions comprise 2 to 4 carbon atoms and the cycloalkyl radicals and portions comprise 3 to 6 carbon atoms, and their salts and their racemates and enantiomers when they comprise at least one asymmetric centre, in the preparation of a medicament intended for the treatment of the abuse of drugs or of substances giving rise to drug addiction or to excessive use, with the exception of the abuse of alcohol, by reducing the self-administration of the said drugs by the patient.

2. Use according to Claim 1, **characterized in that** R represents a methylene radical, a sulphur atom or an SO radical, R₁ represents an optionally substituted phenyl radical, R₂ represents a phenyl or alkoxycarbonyl radical, R₄ and R₅ represent a hydrogen atom and R₃ represents a phenylamino radical, the phenyl ring of which is substituted by a carboxyl, -alk-COOH, -S-alk-COOH, hydroxyalkyl, -alk'-COOH or -alk-SO₃H radical.

3. Use according to Claim 1 or 2, **characterized in that** R₁ and R₂ are in the cis position with respect to one another.

4. Use according to Claim 1, **characterized in that** the compound of formula (I) is chosen from:
- tert-butyl (2RS,5SR)-1-{2-[3-(3-((RS)-1-hydroxyethyl)phenyl)ureido]acetyl}-5-phenylprolinate,
- 2-{3-{3-[2-((2S,5R)-2-(tert-butoxycarbonyl)-5-phenylpyrrolidin-1-yl)-2-oxoethyl]ureido}phenyl}-propionic acid,
- {3-{3-[2-((2RS,5SR)-2-(tert-butoxycarbonyl)-5-phenylpyrrolidin-1-yl)-2-oxoethyl]ureido}phenylthio}acetic acid,
- 2-{3-{3-[2-((2R,4R)-4-(tertbutoxycarbonyl)-2-(2-fluorophenyl)thiazolidin-3-yl)-2-oxoethyl]ureido}-phenyl}propionic acid,
- potassium (RS)-1-{3-{3-[2-((2R,4R)-4-(tert-butoxycarbonyl)-2-phenylthiazolidin-3-yl)-2-oxoethyl]-ureido}phenyl}ethanesulphonate,
- potassium (RS)-1-{3-{3-[2-((2S,5R)-2-(tert-butoxycarbonyl)-5-phenylpyrrolidin-1-yl)-2-oxoethyl]ureido}-phenyl}ethanesulphonate,
- potassium 1-{3-{3-[2-((2S,5R)-2-(tert-butoxycarbonyl)-5-phenylpyrrolidin-1-yl)-2-oxoethyl]ureido}-phenyl}methanesulphonate,
- 3-{3-[2-((2S,5R)-2-(tert-butoxycarbonyl)-5-phenylpyrrolidin-1-yl)-2-oxoethyl]ureido}benzoic acid,
- 3-{3-{2-[(2RS,5SR)-2-(tert-butoxycarbonyl)-5-(2-fluorophenyl)pyrrolidin-1-yl]-2-oxoethyl}ureido}benzoic acid,
- 3-{3-[2-(cis-2,5-diphenylpyrrolidin-1-yl)-2-oxoethyl]ureido}benzoic acid,
- 3-(3-(2-[(2RS, 5SR)-2-(2-hydroxyphenyl)-5-phenylpyrrolidin-1-yl]-2-oxoethyl}ureido}phenylacetic acid,
- 3-{3-[2-((2R,4R)-4-(tert-butoxycarbonyl)-2-phenylthiazolidin-3-yl)-2-oxoethyl]ureido}phenylacetic acid,
- 3-{3-[2-((2R,4R)-4-(tert-butoxycarbonyl)-2-phenylthiazolidin-3-yl)-2-oxoethyl]ureido}benzoic acid,
- 2-{3-{3-[2-((lRS,2R,4R)-4-(tert-butoxycarbonyl)-2-(2-fluorophenyl)-1-oxidothiazolidin-3-yl)-2-oxoethyl]ureido}phenyl}propionic acid,
- 3-{3-[2-((2R,4R)-4-(tert-butoxycarbonyl)-2-(2,3-difluorophenyl)thiazolidin-3-yl)-2-oxoethyl]ureido}-phenylacetic acid,
- tert-butyl (2RS, 5SR)-1-{2-{3-[3-(1-((E)-hydroxyimino)ethyl)phenyl]ureido}acetyl}-5-phenylprolinate.

5. Use according to Claim 4, **characterized in that** the compound of formula (I) is 2-{3-{3-[2-((2R,4R)-4-(tertbutoxycarbonyl)-2-(2-fluorophenyl)thiazolidin-3-yl)-2-oxoethyl]ureido}phenyl}propionic acid.

6. Use according to any one of Claims 1 to 5, **characterized in that** the drug is chosen from nicotine, caffeine, a benzodiazepine, a narcotic or a hallucinogen.

7. Use according to Claim 6, **characterized in that** the narcotic is an amphetamine, cocaine, a cannabinoid, morphine or one of its derivatives, or an opioid.

8. Use according to Claim 6, **characterized in that** the hallucinogen is LSD, ecstasy, mescaline or psilocybin.

## Patentansprüche

1. Verwendung der Verbindungen der folgenden Formel (I): 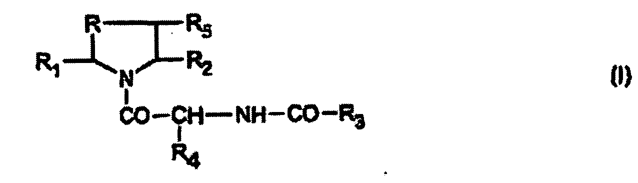 worin
- entweder R einen Methylen-, Ethylen-, SO-, SO₂-, CHOH-Rest oder ein Schwefelatom darstellt, R₁ einen Pyridylrest, der gegebenenfalls mit einem oder mehreren Alkylresten substituiert ist, einen Furylrest, der gegebenenfalls mit einem oder mehreren Alkylresten substituiert ist, einen Thienylrest, der gegebenenfalls mit einem oder mehreren Alkylresten substituiert ist, einen Chinolylrest, der gegebenenfalls mit einem oder mehreren Alkylresten substituiert ist, einen Naphthylrest, der gegebenenfalls mit einem oder mehreren Alkylresten substituiert ist, einen Indolylrest, der gegebenenfalls mit einem oder mehreren Alkylresten substituiert ist, oder einen Phenylrest darstellt, der gegebenenfalls mit einem oder mehreren Substituenten substituiert ist, die ausgewählt sind unter den Halogenatomen und den Resten Alkyl, Alkoxy, Hydroxy, Nitro, Amino, Monoalkylamino, Dialkylamino, Alkoxycarbonyl,
- CO-NR₇R₈, -NH-CO-CH₃, Trifluormethyl oder Trifluormethoxy, und R₅ ein Wasserstoffatom darstellt,
- oder R einen Methylenrest darstellt, R₁ ein Wasserstoffatom darstellt und R₅ einen Phenylrest darstellt,
- oder R einen CHR₆-Rest darstellt, R₁ und R₅ jeweils ein Wasserstoffatom darstellen,
- R₂ einen Alkoxycarbonyl-, Cycloalkyloxycarbonyl-, Cycloalkylalkyloxycarbonyl-,
- CONR₉R₁₀- oder Phenylrest darstellt, der gegebenenfalls mit einem oder mehreren Substituenten substituiert ist, die ausgewählt sind unter den Resten Alkyl, Alkoxy oder Hydroxy,
- R₃ einen Phenylrest (gegebenenfalls substituiert mit einem oder mehreren Substituenten, die ausgewählt sind unter den Halogenatomen und den Resten Alkyl, Alkoxy und Alkylthio), einen Naphthyl-, Indolyl-, Chinolyl-, oder Phenylaminorest darstellt, dessen Phenylkern gegebenenfalls mit einem oder mehreren Substituenten substituiert ist, die ausgewählt sind unter den Halogenatomen und den Resten Alkyl, Alkoxy, Alkylthio, Trifluormethyl, Carboxy, Alkoxycarbonyl, Hydroxy, Nitro, Amino, Acyl, Cyano, Sulfamoyl, Carbamoyl, Hydroxyiminoalkyl, Alkoxyiminoalkyl, Hydroxyaminocarbonyl, Alkoxyaminocarbonyl, 5-Tetrazolyl, 5-Tetrazolyl-alkyl, Trifluormethylsulfonamido, Alkylsulfinyl, Mono- oder Polyhydroxyalkyl, Sulfo, -alk-O-CO-alk, -alk-COOX, -alk-O-alk, -alk'-COOX, -O-alk-COOX, -CH=CH-COOX, -CO-COOX, -alk-SO₃H in Form von Salz, -CH=CH-alk', -C(=NOH)-COOX, -S-alk-COOX, -O-CH₂-alk'-COOX, -CX=N-O-alk-COOX, -alk-N(OH)-CO-alk oder 2,2-Dimethyl-4,6-dioxo-1,3-dioxan-5-yl,
- R₄ ein Wasserstoffatom oder einen Alkylrest darstellt,
- R₆ einen Phenylrest darstellt,
- R₇ ein Wasserstoffatom oder einen Alkyl-, Phenylalkyl- oder Phenylrest darstellt, der gegebenenfalls mit einem oder mehreren Substituenten substituiert ist, die ausgewählt sind unter den Halogenatomen und den Resten Alkyl, Alkoxy und Alkylthio,
- R₈ einen Alkyl-, Phenylalkyl- oder Phenylrest darstellt, der gegebenenfalls mit einem oder mehreren Substituenten substituiert ist, die ausgewählt sind unter den Halogenatomen und den Resten Alkyl, Alkoxy und Alkylthio,
oder aber R₇ und R₈ mit dem Stickstoffatom, an das sie gebunden sind, einen mono- oder polycyclischen, gesättigten oder ungesättigten Heterocyclus mit 4 bis 9 Kohlenstoffatomen und einem oder mehreren Heteroatomen (O, N), der gegebenenfalls mit einem oder mehreren Alkylresten substituiert ist, bilden,
- R₉ ein Wasserstoffatom oder einen Alkyl-, Cycloalkylalkyl-, Cycloalkyl-, Phenylalkyl- oder Phenylrest darstellt, der gegebenenfalls mit einem oder mehreren Substituenten substituiert ist, die ausgewählt sind unter den Halogenatomen und den Resten Alkyl, Alkoxy und Alkylthio,
- R₁₀ einen Alkyl-, Cycloalkylalkyl-, Cycloalkyl-, Phenylalkyl- oder Phenylrest darstellt, der gegebenenfalls mit einem oder mehreren Substituenten substituiert ist, die ausgewählt sind unter den Halogenatomen und den Resten Alkyl, Alkoxy und Alkylthio,
oder aber R₉ und R₁₀ zusammen mit dem Stickstoffatom, an das sie gebunden sind, einen mono- oder polycyclischen, gesättigten oder ungesättigten Heterocyclus mit 4 bis 9 Kohlenstoffatomen und einem oder mehreren Heteroatomen (O, N, S), der gegebenenfalls mit einem oder mehreren Alkylresten substituiert ist, bilden,
- X ein Wasserstoffatom, einen Alkyl- oder Phenylalkylrest darstellt,
- alk einen Alkyl- oder Alkylenrest darstellt,
- alk' einen Hydroxyalkyl-, Hydroxyalkylen-, Alkoxyalkyl- oder Alkoxyalkylenrest darstellt,
ihrer Racemate und Enantiomere, wenn sie ein oder mehrere asymmetrische Zentren enthalten, und ihrer Salze,
wobei es sich versteht, dass die Alkyl-, Alkylen- und Alkoxyreste und die Alkyl-, Alkylen- und Alkoxyteile 1 bis 4 Kohlenstoffatome in gerader oder verzweigter Kette enthalten, die Acylreste und -teile 2 bis 4 Kohlenstoffatome enthalten und die Cycloalkylreste und -teile 3 bis 6 Kohlenstoffatome enthalten, sowie ihrer Salze und ihrer Racemate und Enantiomere, wenn sie wenigstens ein asymmetrisches Zentrum enthalten, für die Herstellung eines Arzneimittels zur Behandlung des Missbrauchs von Drogen oder von Substanzen, die Arzneimittelsucht oder einen übermäßigen Gebrauch verursachen, mit Ausnahme von Alkoholmissbrauch, durch Verringerung der Selbstverabreichung besagter Drogen durch den Patienten.

2. Verwendung gemäß Anspruch 1, **dadurch gekennzeichnet, dass** R einen Methylenrest, ein Schwefelatom oder einen SO-Rest darstellt, R₁ einen gegebenenfalls substituierten Phenylrest darstellt, R₂ einen Phenyl- oder Alkoxycarbonylrest darstellt, R₄ und R₅ ein Wasserstoffatom darstellen und R₃ einen Phenylaminorest, dessen Phenylkern mit einem Carboxy-, -alk-COOH-, -S-alk-COOH-, Hydroxyalkyl-, -alk'-COOH- oder -alk-SO₃H-Rest substituiert ist, darstellt.

3. Verwendung gemäß Anspruch 1 oder 2, **dadurch gekennzeichnet, dass** R₁ und R₂ zueinander in cis-Position stehen.

4. Verwendung gemäß Anspruch 1, **dadurch gekennzeichnet, dass** die Verbindung der Formel (I) ausgewählt ist unter:
- (2RS,5SR)-1-{2-[3-(3-((RS)-1-Hydroxy-ethyl)-phenyl)-ureido]-acetyl}-5-phenylprolinsäure-tert-butylester,
- 2-{3-{3-[2-((2S,5R)-2-tert-Butoxycarbonyl-5-phenyl-1-pyrrolidinyl)-2-oxo-ethyl]-ureido}-phenyl}-propionsäure,
- (2RS,5SR)-{3-{3-[2-(2-tert-Butoxycarbonyl-5-phenyl-1-pyrrolidinyl)-2-oxo-ethyl]-ureido}-phenylthio}-essigsäure,
- 2-{3-{3-[2-((2R,4R)-4-tert-Butoxycarbonyl-2-(2-fluor-phenyl)-3-thiazolidinyl)-2-oxo-ethyl]-ureido}-phenyl}-propionsäure,
- (R,S)-1-{3-{3-[2-((2R,4R)-4-tert-Butoxycarbonyl-2-phenyl-3-thiazolidinyl)-2-oxo-ethyl]-ureido}-phenyl}-ethansulfonat Kaliumsalz,
- (R,S)-1-{3-{3-[2-((2S,5R)-2-tert-Butoxycarbonyl-5-phenyl-1-pyrrolidinyl)-2-oxo-ethyl]-ureido}-phenyl}-ethansulfonat Kaliumsalz,
- (2S,5R)-1-{3-{3-[2-(2-tert-Butoxycarbonyl-5-phenyl-1-pyrrolidinyl)-2-oxo-ethyl]-ureido}-phenyl}-methansulfonat Kaliumsalz,
- (2S,5R)-3-{3-[2-(2-tert-Butoxycarbonyl-5-phenyl-1-pyrrolidinyl)-2-oxo-ethyl]-ureido}-benzoesäure,
- (2RS,5SR)-3-{3-{2-[2-tert-Butoxycarbonyl-5-(2-fluor-phenyl)-1-pyrrolidinyl]-2-oxo-ethyl}-ureido}-benzoesäure,
- (cis)-3-{3-[2-(2,5-Diphenyl-1-pyrrolidinyl)-2-oxo-ethyl]-ureido}-benzoesäure,
- (2RS,5SR)-3-{2-[2-(2-Hydroxy-phenyl)-5-phenyl-1-pyrrolidinyl]-2-oxo-ureido}-phenylessigsäure,
- (2R,4R)-3-{3-[2-(4-tert-Butoxycarbonyl-2-phenyl-3-thiazolidinyl)-2-oxo-ethyl]-ureido}-phenylessigsäure,
- (2R,4R)-3-{3-[2-(4-tert-Butoxycarbonyl-2-phenyl-3-thiazolidinyl)-2-oxo-ethyl]-ureido}-benzoesäure,
- 2-{3-{3-[2-((1RS,2R,4R)-4-tert-Butoxycarbonyl-2-(2-fluor-phenyl)-1-oxid-3-thiazolidinyl)-2-oxo-ethyl]-ureido}-phenyl}-propionsäure,
- (2R,4R)-3-{3-[2-(4-tert-Butoxycarbonyl-2-(2,3-difluor-phenyl)-3-thiazolidinyl)-2-oxo-ethyl]-ureido}-phenylessigsäure,
- (2RS,5SR)-1-{2-{3-[(E)-3-(1-Hydroxyimino-ethyl)-phenyl]-ureido}-acetyl}-5-phenyl-prolinsäure-tert-butylester.

5. Verwendung gemäß Anspruch 4, **dadurch gekennzeichnet, dass** die Verbindung der Formel (1) 2-{3-{3-[2-((2R,4R)-4-tert-Butoxycarbonyl-2-(2-fluorphenyl)-3-thiazolidinyl)-2-oxo-ethyl]-ureido}-phenyl}-propionsäure ist.

6. Verwendung gemäß einem der Ansprüche 1 bis 5, **dadurch gekennzeichnet, dass** die Droge ausgewählt ist unter Nikotin, Koffein, einem Benzodiazepin, einem Rauschmittel oder einem Halluzinogen.

7. Verwendung gemäß Anspruch 6, **dadurch gekennzeichnet, dass** das Rauschmittel ein Amphetamin, Kokain, ein Cannabinoid, Morphium oder eins seiner Derivate oder ein Opiat ist.

8. Verwendung gemäß Anspruch 6, **dadurch gekennzeichnet, dass** das Halluzinogen LSD, Ecstasy, Meskalin oder Psylocibin ist.
